# EUROPEAN PATENT APPLICATION

(11) **EP 1 736 180 A1**
(43) Date of publication of application: **27.12.2006**
(21) Application number: 05719070.4
(22) Date of filing: 14.02.2005
(51) Int. Cl.: A61L 27/00, C12N 5/06

(54) **CORNEAL EPITHELIAL SHEET, METHOD OF CONSTRUCTING THE SAME AND TRANSPLANTATION METHOD USING THE SHEET**

(30) Priority: 11.03.2004 JP 2004069145
(71) Applicant: ArBlast Co., Ltd., Kobe Hyogo 650-0047 (JP)
(72) Inventor: HASHIMOTO, Kouji, Ehime 7910314 (JP); SHIRAKATA, Yuuji, 7910204 (JP); OHASHI, Yuuichi, 6-chome, Matsuyama-shi, Ehime 7900833 (JP); HAMURO, Junji, Kanagawa 2410813 (JP)
(74) Representative: Müller Fottner Steinecke
(86) International application number: PCT/JP2005/002123
(87) International publication number: WO 2005/087285

(57) **Abstract**

A corneal epithelial sheet having potential for the achievement of a favorable therapeutic effect and being highly safe in transplantation. The corneal epithelial sheet is constructed by: (a) preparing corneal epithelial cells; (b) separately culturing human fibroblasts in a collagen gel; (c) sowing or placing the corneal epithelial cells on the collagen gel; and (d) culturing and proliferating the corneal epithelial cells in the absence of xenogeneic animal cells.

## Description

### TECHNICAL FIELD

The present invention relates to a corneal epithelial sheet. More specifically, the present invention relates to a corneal epithelial sheet constructed by culturing and proliferating corneal epithelial cells on a collagen gel without using cells derived from xenogeneic animals as feeder cells, a method of constructing the sheet and use (a transplantation method, and the like) of the sheet.

### BACKGROUND ART

The cornea is one of the tissues to which regenerative medicine is expected to contribute. The cornea is located in the outermost layer of the optical system constituting the eyeball and is a transparent tissue normally having no blood vessels. The cornea contributes to obtaining a good visual acuity by forming a smooth surface along with tear. Furthermore, keratoconjunctival epithelial cell is usually brought into contact with the outside and has an effect of protecting the eyeball from foreign objects such as microorganism in the outside, ray such as ultraviolet ray, and the like. That is to say, the keratoconjunctival epithelial cells play an extremely important role of protecting the transparency of the corneal and the entire eyeball so as to maintain homeostasis.

The cornea may lose its transparency by conditions such as keratitis, cornea ulcer, punch, and the like, and the transparency may be lost. With respect to the permanent deterioration of visual acuity due to the opacificity of cornea, treatment of transplanting a cornea that has been supplied from a donor of the eyeball is carried out. The transplantation of the cornea is carried out by transplanting the transparent cornea after removing the patient's cornea whose transparency has been lost. This transplantation recovers the transparency and enables the visual acuity to be recovered again.
Although such cornea transplantation offers an effective treatment effect, there are some diseases that cannot be treated only by transplantation of the cornea. An example of such diseases includes Stevens-Johnson syndrome, ocular pemphigoid, chemical injury, burn, and the like. In general, the keratoconjunctival epithelial cell divides every day, and old cells are peeled off and new cells are regenerated from the stem cell tissue. However, it has come to be reported that in the above-mentioned conditions, the stem cell tissue for regenerating the cornea has the defect in the potency to regenerate corneal tissue.

The stem cell tissue for regenerating the corneal epithelium is referred to as "corneal limbus tissue" and localized in the boundary portion between the black and white eye and in the specific environment exposed to the outside. In the above-mentioned pathologic conditions, it is thought that this stem cell tissue itself undergoes some defects and become deficient. Then, due to this deficiency of the stem cell tissue, the defective portion is covered with the conjunctiva epithelium existing around the defective portion. Thus, the transparency is lost, resulting in extreme deterioration of the visual acuity. In this way, in the above-mentioned pathologic conditions, since the corneal limbus is deleted, even if only the cornea is transplanted, the transplanted cornea cannot be maintained for a long term. Therefore, in order to reconstruct the ocular surface permanently, it is necessary that the corneal limbus is also transplanted. As one of the methods of transplanting this corneal limbus, a method of transplanting amniotic membrane has been developed (see Medical Asahi, September, 1999: p62-65, N Engl J Med 340: 1697 to 1703, 1999: non patent document 1). Amniotic membrane to be used for this transplantation can be obtained from the placenta of, for example, a pregnant woman who underwent caesarean section. Since amniotic membrane has thick basal membrane, when it is transplanted, it acts as a substrate on which the keratoconjunctival epithelial cells proliferate and differentiate. Amniotic membrane hardly has immunogenicity. In addition, amniotic membrane has effects such as anti-inflammation and suppression of cicatrisation. The keratoconjunctival epithelium and the stem cells thereof, which are transplanted on the amniotic membrane, are free from rejection of a transplantation recipient.

[Non-patent document 1] Medical Asahi, September, 1999: p62-65, N Engl J Med 340: 1697 to 1703, 1999

### SUMMARY OF THE INVENTION

### [Problems to be Solved by the Invention]

As surgical treatment for ocular surface diseases in which the cornea is covered with conjunctival epithelium and becomes non-transparent, at present, cornea epithelium transplantation is carried out. However, in refractory keratoconjunctival diseases with strong inflammation (Stevens-Johnson syndrome, ocular pemphigoid, corneal erosion, and the like), the prognosis is extremely bad. The prime reason is that allogenic (allo) cornea epithelium having strong antigenicity is recognized and rejected by an immune system of a host. Furthermore, a complication caused by systemic or local application of a large amount of immunosuppressant agent after operation for prevention of rejection reaction is also a critical factor of unfavorable prognosis. On the other hand, use of allo cornea epithelium has a problem of shortage of the number of the donors. When the technology capable of constructing several tens of cornea sheets by using cornea obtained from one eye is realized, the problem of shortage of the number of the donors can be solved.

### [Means to Solve the Problems]

In view of the above-mentioned circumstances and problems, the present invention was made. The objective of the present invention is to provide a corneal epithelial sheet by which high treatment effect can be expected and which offers high degree of safety in transplantation. In order to achieve such a objective, the present inventors have attempted to produce a corneal epithelial sheet under the conditions that in view of safety, cells (feeder cells) derived from xenogeneic animals are not used when epithelial cells are cultured. As a result, by culturing corneal epithelial cells on a collagen gel containing human fibroblasts, stratification and the formation of epithelium can be achieved without using a feeder cell. In particular, by using a serum free medium, favorable results can be obtained.
As mentioned above, the present inventors have succeeded in constructing safe and practical corneal epithelial sheet without using cells derived from xenogeneic animals at all.

The present invention was made based on the above-mentioned results and findings and provides the following configurations.
[1] A corneal epithelial sheet comprising corneal epithelial cells proliferated on a collagen gel containing human fibroblasts in the absence of a xenogeneic animal cell.
[2] The corneal epithelial sheet according to [1], in which the corneal epithelial cells are proliferated by using a serum free medium.
[3] The corneal epithelial sheet according to [1], in which the corneal epithelial cells are proliferated by using a medium containing only serum derived from a recipient as a serum component.

### EFFECT OF THE INVENTION

The corneal epithelial sheet of the present invention has extremely high safety because cells derived from heterogeneous animals are not used in the production process. Furthermore, by using a collagen gel containing fibroblasts, an extremely dense cell layer is formed. Thus, stratification culture becomes possible for the first time and survival after transplantation is extremely enhanced.

### BRIEF DESCRIPTION OF THE DRAWINGS

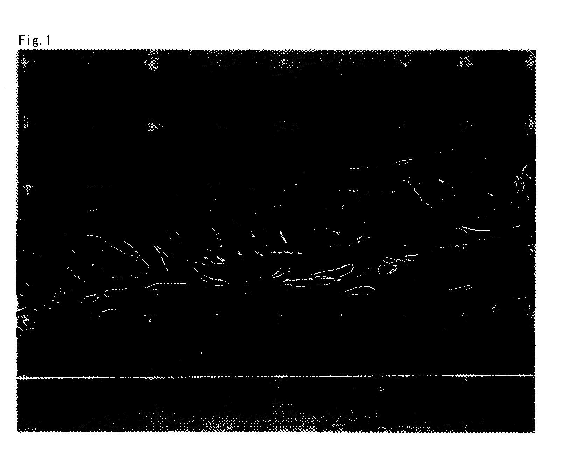

Fig. 1 shows a hematoxylin- and eosin- stained image of a corneal epithelial sheet constructed by the method described in Example. Three to four layers of corneal cell layers are observed on gel containing fibroblasts. The form of cell is similar to that of normal cornea and on the top layer, the formation of a horny cell layer is not observed, showing a form of the cornea.

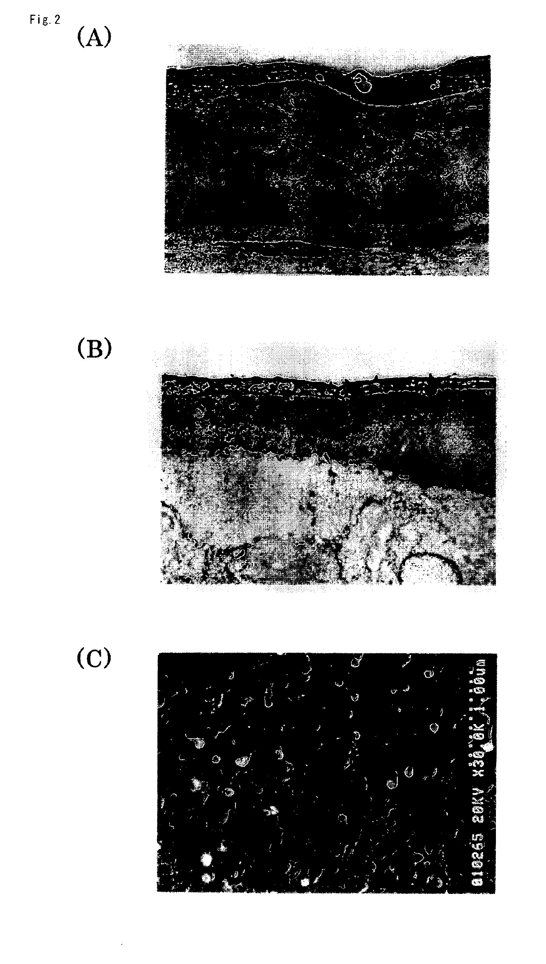

Fig. 2 shows electron microscopic images of the corneal epithelial sheet constructed by the method described in Example. The corneal epithelial cell layer includes three to four layers of cells (A). In the basal membrane portion, the formation of hemidesmosome and lamina densa is observed (B). In an intercellular portion, desmosome is well formed (C). In the top layer, microvilli are formed, showing substantially the same findings as those of electron microscopy findings of the human cornea. Thus, it is thought that a micro structure also has the form of cornea.

### BEST MODE FOR CARRYING OUT THE INVENTION

The present invention provides a corneal epithelial sheet having the following structure. That is to say, the present invention provides a corneal epithelial sheet comprising a cell layer of corneal epithelial cells proliferated on a collagen gel in the absence of a xenogeneic animal cell. The corneal epithelial sheet of the present invention is used for regenerating (reconstructing) corneal epithelium. Hereinafter, the corneal epithelial sheet and the method of constructing the same are described in detail.

The corneal epithelial sheet is constructed by a method including the steps of: (a) preparing corneal epithelial cells; (b) culturing human fibroblasts in a collagen gel; (c) plating or placing the corneal epithelial cell on the collagen gel; and (d) culturing and proliferating the corneal epithelial cells in the absence of a heterogeneous animal cell.
In the step a, corneal epithelial cells are prepared. Unless otherwise specified, "corneal epithelial cells" in this specification is intended to mean cells contained in a corneal epithelial cell layer inclusively. That is to say, the corneal epithelial cell also includes a stem cell and a precursor cell thereof. One example of the method for preparing the corneal epithelial cells is described hereinafter.

Corneal epithelial cells can be obtained from a corneal limbus tissue. For example, endothelial cells are peeled off and removed from corneal limbus tissue, and conjunctiva is excised so as to form a single cell suspension. Then, this is preserved in a nitrogen tank, and then rapidly melted at 37°C so as to prepare a corneal epithelial cell suspending solution. If necessary, subculture is carried out. For subculture, for example, EpiLife™ (Cascade Biologics), an MCDB153 medium (NISSUI PHARMACEUTICAL CO., LTD.), which are serum free media, and medium produced by modifying the amino acid composition. etc. of these media can be used.

It is preferable that the corneal epithelial cells are prepared from a person (recipient) who undergoes transplantation of the corneal epithelial sheet. That is to say, it is preferable that a donor of the corneal epithelial cells is identical to a recipient of the corneal epithelial sheet. By using such autologous cells, unfavorable immunological rejection is avoided.

In the step b, human fibroblasts are cultured in a collagen gel. "Collagen gel" functions as a culture substrate of human fibroblasts. The kinds of collagens as a material of the collagen gel are not particularly limited, and type I collagen, type III collagen, and type IV collagen, and the like, can be used. A plurality of collagens can be used in combination thereof. Such collagens can be extracted and purified from the connective tissue of the skin and cartilage, etc. of animals such as swine, bovine, sheep, etc., by an acid solubilization method, alkali solubilization method, and oxygen solubilization method, and the like. Note here that for the purpose of deteriorating the antigenicity, it is preferable that a so-called atherocollagen obtained by removing telopeptide by a treatment with the use of catabolic enzyme such as pepsin, trypsin, etc. As materials of the collagen gel, a collagen derived from amniotic membrane, particularly derived from human amniotic membrane may be used. Herein, the collagen layer is "derived from amniotic membrane" broadly means that the collagen gel is obtained by using amniotic membrane as a starting material.

The origin of the human fibroblast contained in the collagen matrix is not particularly limited and it may be derived from any tissue as long as the tissue produces collagen. Human fibroblasts prepared from, for example, skin tissue, oral mucosa tissue, and the like, can be used. '

A specific example of the method of forming a collagen matrix is shown. Firstly, fibroblasts are prepared by the following procedure. The skin is collected, and then dermis is peeled off from the skin. The dermis is cut in strips and is brought into close contact with a dish coated with type I collagen. After static culture, fibroblasts migrated from the dermis strip are subcultured. Cells are peeled off from the bottom surface of the dish so as to prepare a cell suspension, which is sowed on a cell culture dish. Appropriately, cells arc cryopreserved (for example, stored in liquid nitrogen).
On the other hand, a neutralized collagen solution is prepared using type I collagen (see the below-mentioned Example). This is added in a culture container (for example, a culture insert) and stood still for ten minutes at room temperature so as to be gelled. Next, fibroblasts in a logarithmic growth phase, which has been cultured by the above-mentioned method in advance, is mixed with this gel and gelled again. Thereafter, static culture is carried out. A collagen matrix containing fibroblasts can be obtained by the above-mentioned procedure. This inventiveness allows the collagen matrix to have necessary strength and to have amniotic membrane layer or cells of biological origin to be mounted thereon, which forms a base of the present invention. A separately prepared corneal epithelial cells can be sowed (placed) on the collagen matrix.
The corneal epithelial cells are plated so that, for example, the cell density becomes about 1×10³ cells/cm² or more, preferably in the range from about 1×10³ cells/cm² to about 1×10⁷ cells/cm², furthermore preferably, in the range from about 1×10⁴ cells/cm² to about 1×10⁶ cells/cm².

Culturing of the corneal epithelial cells plated on the collagen matrix (the step d) is carried out in the absence of a xenogeneic animal cell. In the present invention, "in the absence of a heterogeneous animal cell" means that cells of animals that are different kinds form those of the corneal epithelial cells are not used as conditions for culturing corneal epithelial cells. Specifically, the conditions include that when human cells are used as corneal epithelial cells, cells from non-human animals such as mouse, rat, and the like, do not exist (coexist). By culturing under such a condition, finally obtained transplantation materials (that is, biological tissue sheet) may not contain components of xenogeneic species origin (including xenogeneic cells themselves).
The medium to be used for culturing corneal epithelial cells is not particularly limited as long as it allows the cells to proliferate. In particular, in the present invention, it is preferable to use a medium that is serum free and does not contain protin derived from heterogeneous animals. On the other hand, a medium to which a growth factor, antibiotics, and the like, are added may be used. However, a medium that does not contain serum is preferable. That is to say, in a culturing method of the present invention, it is preferable to employ a serum free culture method. This is advantageous because a problem such as immunological rejection caused by contamination of components derived from serum can be avoided. Note here that culturing may be carried out in a medium containing serum, however in this case, it is preferable to use serum of the same species origin (when human corneal epithelial cells are cultured, serum of human origin is used) or to use autologous serum. Of course, if possible, it is preferable that autologous serum that may not cause immunological rejection.
For the purpose of proliferating corneal epithelial cells well, culturing conditions can be changed in the middle of the culture step.

As a result of the step d, corneal epithelial cells proliferate on the collagen matrix. In order to promote keratinization of the thus obtained cell layer, it is preferable that a step of bringing the surface layer of the cell layer into contact with air (the step e) is carried out.
Note here that this step is referred to as air-lifting in this specification. This step e is carried out in order to differentiate the cells forming a cell layer and induce a barrier function.
This step can be carried out by temporarily removing a part of a culture solution by using a dropping syringe, a pipette, and the like, so as to lower the surface of the culture solution, and thus temporarily exposing the outermost layer of the cell layer to the outside of the culture solution. Alternatively, this step can be carried out by lifting the cell layer included in the collagen matrix so as to temporarily expose the outermost layer from the surface of the culture solution. Furthermore, by sending the air into the culture solution by using a tube, etc., the outermost layer of the cell layer may be brought into contact with the air. From the viewpoint of ease in operation, it is preferable that the outermost layer of the cell layer is exposed by towering the surface of the culture solution.
The duration of carrying out this step e, that is, the time for which the outermost layer of the cell layer is brought into contact with the air changes depending upon the state of cells and culture conditions. However, for example, it is about three days to three weeks, preferably, five days to two weeks, and more preferably about one week.
Hereinafter, the present invention is described specifically with reference to Example, however, the present invention is not necessarily limited to the following Example.

### [Example 1]

### 1. Preparation of corneal epithelial cell

### 1-1. Procurement of cornea

Donor corneas were purchased from Northwest Lions Eye Bank (Seattle, USA).

### 1-2. Serum free culture method of corneal epithelial cells

Cornea is transferred to a petri dish containing Dulbecco's phosphate buffer (PBS) and the limbus is cut into strip with the size of 2 to 3 mm × 2 to 3 mm by using a surgical knife under stereoscopic microscope. The limbus strip is washed with PBS several times and was sterilized by dipping it into 70% ethanol for one minute. The strip is washed with PBS, dipped in Dispase solution (Dispase II, Goudou Shusei, 250 units/ml, Dulbecco's Modified MEM medium; DMEM) and stood still overnight (18 to 24 hours) at 4°C. On the following day, by using forceps, epithelium is peeled off from the substance under stereoscopic microscope. The peeled off corneal epithelium is washed with DMEM, then washed with PBS, and dipped into 0.25% trypsin solution to carry out treatment at 37°C for 10 minutes. Epidermis is transferred to a plastic petri dish containing a trypsin neutralization solution, disentangled by using forceps, and transferred to 15 ml sterilization tube. PBS is added to prepare a corneal epithelial cell suspending solution. The number of cells is counted and the cells are subjected to centrifugation at 1000 rpm for 5 minutes, so that the cells are precipitated. Supernatant is sucked and the cells are suspended in an EpiLife medium that is a serum free medium, which is sowed at the rate of 1 to 2 × 10⁶ cells / 5 ml culture solution for each 60 mm petri dish coated with collagen (ASAHI TECHNO GLASS CORPORATION, type I collagen coated dish; 4010-020). On the following day, the culture solution is exchanged, and later than that day, the culture solution is exchanged every other day. At the time when the cell density becomes about 70% to 80%, subculture is carried out.
Note here that in the above-mentioned method, by using a serum free medium, corneal epithelial cells are cultured. However, a medium containing serum can be used as in the following procedures.
(1) Peel and remove endothelium cells from the corneal limbus tissue and excise conjunctiva.
(2) Dip in Dispase solution (Dispase I, Goudou Shusei, 250 units/ml, Dulbecco's Modified MEM medium; DMEM) and stand it still overnight (for 18 to 24 hours) at 4°C.
(3) Dip in a 0.25% trypsin Solution and treat at 37°C for 10 minutes.
(4) Peel only epithelium in a trypsin solution under microscope.
(5) Carry out pipetting and add the same amount of 30% FCS/DMEM so as to obtain suspension.
(6) Collect the remaining cells by PBS (-) and carry out centrifugation.
(7) Use a proper amount of culture solution so as to obtain a single cell suspension.

An example of cryopreservation conditions (including the composition of a stock solution) and melting conditions of the prepared corneal epithelial cells is shown bellow.
Cryopreservation conditions: lower the temperature to -20°C at the rate of 1°C/hour and then preserve in a nitrogen tank.
Composition of stock solution: 20% FCS /10% DMSO / DMEM
Melting conditions: melt at 37°C as quickly as possible and dilute 10 times with PBS.

### 2. Preparation of fibroblasts

After washing with DMEM, the peeled dermis is cut into strips with the size of 1 to 2 mm × I to 2 mm by using a surgical knife. The cut dermis strip is brought into close contact with a dish coated with type 1 collagen at intervals of about 1 cm. Then, the dermis is stood still in a CO₂ incubator for 30 minutes so as to be brought into close contact the dish completely. Thereafter, about 5 ml of DMEM medium containing 10% fetal bovine serum is added and stood still for seven days. On day 7, initial exchange of the culture solution is carried out. It is confirmed that fibroblasts are migrated from the dermis strip. At the stage when cells are proliferated and migrated to 5 mm vicinity of the dermis strip, subculture is carried out. The dermis is washed with PBS, and then a solution containing 0.125% trypsin and 0.05% EDTA is added and treated at 37°C for three minutes. After it is confirmed through a microscope that cells are detached from the bottom surface of the dish, 3 ml trypsin inhibitor is added and the cells are collected and transferred to 50 ml tube. By using PBS, remaining cells are collected and subjected to centrifugation at 1000 rpm for five minutes, so that cells are precipitated. The supernatant is sucked, and then a DMEM medium containing 10% fetal bovine serum is added so as to prepare a cell suspending solution, which is plated on a cell culture dish. The cell density of subculture is about 1 : 3. The cells are cryopreserved appropriately. As a cryopreservation solution, 10% glycerol, 20% FCS and 70% DMEM are used, and stored in liquid nitrogen.

### 3. Preparation of neutralized collagen gel

A neutralized collagen solution (final concentration of collagen: 1 mg/ml) is produced at 4°C by mixing one volume of 0.1N NaOH, one volume of 8 times concentration DMEM, ten volumes of 20% FCS/DMEM to six volumes of type I collagen solution (cell matrix type 1A: 3 mg/ml: Nitta Gelatin Inc.). One ml each of the neutralized collagen solution is dropped into 24 mm diameter culture insert (Corning-Costar) and stood still at room temperature for 10 minutes so as to be gelled. Fibroblasts in a logarithmic growth phase, which has been prepared in advance (cells are subjected to Dispase treatment to peel off epidermis and the remaining dermis is subcultured for 5-10 generations by an outgrowth method, and thus the subcultured cells are used) are adjusted to the concentration of 5×10⁵ cells/ml and 10% FCS/DMEM. This cell suspension (2 volumes) is mixed with a neutralized collagen solution (8 volumes) so as to prepare a neutralized collagen solution containing cells (final concentration of collagen: 0.8 mg/ml). To each culture insert, 3.5 ml each of this solution is added, and the culture insert is stood still in a CO₂ incubator (37°C, 5% CO₂), After 30 minutes, it is confirmed that the solution is gelled. Thereafter, 10% FCS/DMEM is added so that gel is dipped therein (3 mi is added to the inside of the culture insert, and 3 ml is added to the outside of the culture insert) and static culture is carried out for five days. On day 2 after culture is started, the gel starts to shrink. The proliferation of fibroblasts can be observed under phase contrast microscope.

### 4. Plating of corneal epithelial cell

On day 5 after culturing of fibroblasts is started, the bottom surface of the collagen gel is attached to the membrane. Meanwhile, the upper part of the collagen gel is shrunk so that diameter is about 13 to 15 mm and thickness is about 2 to 3 mm. The middle portion of the collagen gel is depressed in a shape of crater. On this depressed portion, corneal epithelial cells are plated. That is to say, the corneal epithelial cells prepared in 1-2 are detached from the dish and collected by using trypsin - EDTA. The corneal epithelial cells are subjected to centrifugation at 1000 rpm for five minutes to remove the supernatant so as to adjust to about 3 to 4×10⁶ cells/ml. Suspension (volume: 50-60 µl) can be added to the collagen gel and stood still in a CO₂ incubator for 1.5 to 2.0 hours so that corneal epithelial cells are brought into close contact with the amniotic membrane. Thereafter, medium for proliferating epidermal cells is gently added (3 ml to the inside of the culture insert and 3 ml to the outside of the culture insert). Culture is continued for further 14 days in the liquid phase. On day 3 following the sowing of corneal epithelial cells, the culture solution is exchanged with a culture solution for stratification (see below), and later than that day, culture solution is exchanged every other day.
The medium for stratification is prepared as follows. Dulbecco's Modified MEM medium: F-12 medium = I : 1, calcium concentration; 1.95 mM, monoethanolamine; 0.1 mM, 0-phosphoethanolamine; 0.1 mM, insulin; 5 ug/ml, hydrocortisone; 0.4 ug/ml, L-glutamine; 4mM, Adenin; 0.18 mM, transfferin; 5 ug/ml, selenious acid; 53 nM, triiodothyronine; 20 pM, serine; 1 mM, choline chloride; 0.64 mM, linoleic acid; 2 ug/ml, and FCS; 2%.

### 5. Culture under vapor phase conditions

On day 14 following the sowing of corneal epithelial cells, air exposure (air lifting) is carried out. Sterilized filter paper is set to a maintaining vessel for air exposure, a stratifying medium is added so that the filter paper is dipped (about 9 ml). The culture solution inside the culture insert is carefully removed and the culture insert is transferred onto the filter paper and cultured in a CO₂ incubator. On day 3, the culture solution is exchanged. By air exposure for 3 days, a cultured cornea is completed.

### 6. Histological analysis

On day 3 following the air exposure, corneal epithelium has 3 to 4 layers and the formation of horny cell layer is observed, showing substantially the same structure as that of the normal human cornea (see Figs. 1 and 2).

### INDUSTRIAL APPLICABILITY

A corneal epithelial sheet provided by the present invention is used for regenerating (reconstructing) of corneal epithelium. Furthermore, the corneal epithelial sheet of the present invention can be used for gene therapy. The gene therapy is largely classified into in vivo method and ex vivo method. In the in vivo method, gene is directly introduced into the living body and in the ex vivo method, once a cell is taken out, gene is introduced the cell and the cell is returns to the body again. In the view of the current state of the art of gene therapy, only by introducing a gene into cultured corneal epithelium, the ex vivo method can be carried out immediately. The effectiveness of gene introduction to keratinocytes by using various virus vectors has been shown. In particular, when an adenovirus vector is used, gene can be introduced into substantially 100% of keratinocytes.

The present invention is not limited to the description of the above embodiments and Examples. A variety of modifications, which are within the scopes of the claims and which can be easily achieved by a person skilled in the art, are included in the present invention.
All of the articles, publication of unexamined patent application, and Patent Gazette cited herein are incorporated in their entirety by reference.

## Claims

1. A corneal epithelial sheet comprising corneal epithelial cells proliferated on a collagen gel containing human fibroblasts in the absence of a xenogeneic animal cell.

2. The corneal epithelial sheet according to claim 1, wherein the corneal epithelial cells are proliferated by using a serum free medium.

3. The corneal epithelial sheet according to claim 1, wherein the corneal epithelial cells are proliferated by using a medium containing only serum derived from a recipient as a serum component.

4. A method of constructing a corneal epithelial sheet, the method comprising the following steps:
(a) preparing corneal epithelial cells;
(b) culturing human fibroblasts in a collagen gel;
(c) plating or placing the corneal epithelial cells on the collagen gel; and
(d) culturing and proliferating the corneal epithelial cells in the absence of a xenogeneic animal cell.

5. The constructing method according to claim 4, further comprising the following step:
(e) after the corneal epithelial cells are proliferated, bringing the outermost surface layer into contact with air.

6. The constructing method according to claim 4 or 5, wherein the step (d) is carried out by using a serum free medium.

7. The constructing method according to claim 4 or 5, wherein the step (d) is carried out by using a medium containing only serum derived from a recipient as a serum component.

8. A transplantation method using the corneal epithelial sheet according to any one of claims I to 3 as a transplantation material.
